# FASCICULE DE BREVET EUROPEEN

(11) **EP 0 296 937 B1**
(45) Date de publication et mention de la délivrance du brevet: **03.03.1993**
(21) Numéro de dépôt: 88401482.0
(22) Date de dépôt: 15.06.1988
(51) Int. Cl.: C07C 227/38

(54) **Procédé de séparation d'acides aminés**
Verfahren zur Trennung von Aminosäure
Process for the separation of amino acids

(30) Priorité: 16.06.1987 FR 8708356
(43) Date de publication de la demande: 28.12.1988
(73) Titulaire: LABORATOIRES FLORK S.A., 63100 Clermont Ferrand (FR)
(72) Inventeur: Flork, Michel Louis André, F-63400 Chamalières (FR)
(74) Mandataire: Rinuy, Santarelli

(56) Documents cités:
- DE-C- 3 326 633
- FR-A- 2 567 413
- US-A- 2 894 954
- J.P. GREENSTEIN et al.: "Chemistry of the Amino Acids", vol. 2, pages 1452-1461, John Wiley & Sons, Inc., New York, US
- INDUSTRIAL AND ENGINEERING CHEMISTRY, vol. 41, no. 3, mars 1949, pages 460-463, Washington, D.C., US; J.C. WINTERS et al.: "10N exchange in the pharmaceutical field"

## Description

La présente invention a pour objet un procédé de séparation au moyen de résines échangeuses d'ions d'un mélange d'acides aminés provenant notamment de l'hydrolyse acide de protéines.

Il est connu depuis longtemps d'utiliser des résines anioniques ou cationiques, c'est-à-dire échangeuses respectivement d'anions ou de cations, pour séparer des acides aminés présents dans une solution en jouant sur leurs différents points isoélectriques.

C'est ainsi que, dès 1945, les brevets US-A-2.386.926 et 2.387.824 de Richard J. BLOCK préconisent la séparation des acides aminés basiques, arginine, lysine et histidine par passage d'un hydrolysat de protéines les contenant sur une résine cationique qui fixe ces acides aminés et que le brevet US-2.480.654 de MERCK recommande de les récupérer par élution à l'ammoniaque.

De même, l'article de WINTERS et autre paru dans "Industrial and Engineering Chemistry, 41, P. 460-463, et le passage de l'ouvrage de GREENSTEIN qui s'y réfère ("Chemistry of the Amino-Acids", Vol.2, P. 1.455), prévoient le passage successif d'un melange d'acides aminés sur une résine anionique puis sur une résine cationique, en vue de la rétention sélective de certains d'entre des acides mais aucun de ces deux documents d'art antérieur ne décrit ou n'envisage le recyclage de mélange d'acides aminés sur les mêmes résines échangeuses d'ions.

Cependant si le recours à des colonnes chargées de résines échangeuses d'ions a été envisagé très tôt pour scinder en plusieurs fractions un hydrolysat contenant de nombreux acides aminés, ces procédés se sont heurtés à un certain nombre de difficultés qui n'ont pas permis leur utilisation à l'échelle industrielle.

En effet les colonnes devant fixer un acide aminé déterminé ne le font qu'en partie et en laissant passer une proportion non négligeable, appelée fuite; d'autre part étant donné la proximité des constantes d'acidité des couples acide-base entrant dans la composition des acides aminés, la séparation d'un acide-aminé des autres acides aminés n'est jamais parfaite; de plus les proportions d'acide aminé fixé par volume de résine échangeuse d'ions ne sont pas suffisantes pour envisager leur emploi en dehors d'un laboratoire et certains acides aminés très peu solubles dans l'eau, comme la cystine, cristallisent dans les colonnes.

Seuls des progrès de détail ont pu être réalisés pour améliorer le fractionnement d'un mélange d'acides aminés à l'aide de ces résines.

Par exemple le brevet DE-C-3.326.633 de KALI-CHEMIE, publié en 1985, précise des modes opératoires permettant d'extraire à partir d'un hydrolysat de protéines neutralisé sur résine anionique faible, soit une fraction Arg-Lys-His par passage sur une résine cationique forte soit une fraction Arg-Lys sans His par passage sur la résine cationique forte sous forme ammonium d'une partie seulement de l'hydrolysat neutralisé, à savoir celle sortant de la colonne de neutralisation à un pH compris entre 6 et 8.

La présente invention a pour objet d'effectuer de manière beaucoup plus complète la séparation des acides aminés d'un mélange les contenant en utilisant au maximum les possibilités de fixation de certains d'entre eux par passages de ce mélange sur une série de colonnes remplies soit de matière absorbante ne présentant pas de propriétés d'échange d'ions soit de résines échangeuses d'ions et sans addition de réactifs extérieurs à la solution de départ contenant le mélange d'acides aminés à séparer.

Lors du passage, appelé aussi percolation, de la phase aqueuse contenant le mélange d'acides aminés sur une colonne qui contient un produit retenant une partie d'entre eux, la composition du mélange est modifiée, tant du point de vue de sa force ionique que de ses constituants basiques et acides, qui peuvent ou non former un milieu tampon selon son pH.

En jouant non seulement sur le choix des matières contenues dans les colonnes où passe la solution de départ à traiter mais également sur les différents passages de cette solution sur ces colonnes, on a trouvé, par expérimentation, un procédé qui permet de séparer successivement, en groupe ou individuellement, la totalité des acides aminés entrant dans la composition de la solution initiale.

Le procédé selon l'invention vise à séparer, au moyen de résines échangeuses d'ions, des acides aminés en mélange dans une solution aqueuse par passage de la solution sur une colonne de résine anionique puis une colonne de résine cationique, ou inversement.

Il se caractérise par au moins un renouvellement de passage de la solution sur ce même couple des mêmes colonnes disposées dans le même ordre et le fait que les passages successifs sur les colonnes se font sans aucun apport de substances nouvelles, l'évolution de l'action sélective d'une colonne résultant uniquement de la modification de la composition de la solution provoquée par l'échange d'ions sur la colonne antagoniste.

En d'autres termes le procédé selon l'invention consiste à faire passer successivement la solution du mélange d'acides aminés à séparer sur une colonne remplie de résine anionique et sur une colonne remplie de résine cationique puis à nouveau au moins une fois sur la même colonne de résine anionique et enfin sur la même colonne remplie de résine cationique.

Selon une autre caractéristique, ce procédé comprend également le passage, au préalable, de la solution du mélange d'acides aminés à séparer sur des colonnes disposées en série et comprenant successivement au moins une colonne remplie de matière absorbante, ensuite au moins une colonne remplie de résine anionique ou cationique et enfin au moins une colonne remplie de résine cationique si la colonne précédente est remplie de résine anionique ou, au contraire, une colonne remplie de résine anionique si la colonne précédente est remplie de résine cationique, la totalité de la solution ayant traversé la premiere colonne servant à l'alimentation de la colonne suivante et ainsi de suite.

Selon encore une autre caractéristique, le procédé selon l'invention consiste à soumettre au moins une et de préférence chacune des colonnes recevant la solution du mélange d'acides aminés à séparer, qu'il s'agisse d'une colonne remplie de matière absorbante ou remplie de résine anionique ou cationique, à un traitement préalable par un agent oxydant.

Dans le cadre du présent mémoire descriptif, on entend par matière absorbante une matière se présentant sous forme de particules ayant une grande surface active favorisant l'absorption physique de produits fluides avec lesquels elle est en contact et ne présentant pas de propriétés d'échange d'ions, par exemple du charbon actif granulé; on entend par résine anionique une résine échangeuse d'anions donc constituée de cations, par exemple des résines à base forte du type à ammonium quaternaire ou des résines à base faible du type à amine tertiaire, et enfin on entend pur résine cationique une résine échangeuse de cations donc constituée d'anions, par exemple des résines à acide forts comme les acides phényl sulfonique ou méthylène sulfonique ou des résines à acide faible comme les acides carboxyliques ou les phénols.

L'utilisation d'au moins une colonne remplie de matière absorbante en amont d'un système comprenant au moins deux colonnes échangeuses d'ions dont l'une est remplie de résine anionique et l'autre de résine cationique constitue une caractéristique importante de la présente invention.

En effet, il a été trouvé qu'au cours de cette première étape de percolations successives en continu du mélange d'acides aminés à séparer, la ou les colonnes de matière absorbante situées en tête du dispositif non seulement retiennent les acides aminés aromatiques comme la tyrosine et la phénylalanine, mais permettent également la fixation ultérieure de quantités plus importantes d'acides aminés sur les colonnes suivantes remplies de résines échangeuses d'ions, comme il sera mentionné plus en détails ci-après.

Après son passage sur la colonne remplie de matière absorbante, la solution aqueuse du mélange d'acides aminés à séparer passe d'abord sur une colonne remplie de résine cationique et ensuite sur une colonne remplie de résine anionique si cette solution aqueuse est basique, ce qui est le cas notamment des jus sucrés de l'industrie de la betterave ou, au contraire, elle passe d'abord sur une colonne remplie de résine anionique et ensuite sur une colonne remplie de résine cationique si cette solution aqueuse est acide, ce qui est le cas notamment des jus d'hydrolyse des protéines.

Dans ce dernier cas, la ou les colonnes de résines cationiques, qui travaillent alors en queue du dispositif, retiennent les acides aminés basiques comme l'arginine, la lysine et l'histidine.

Après le prélèvement du premier ou de préférence de ces deux groupes d'acides aminés dans le mélange à séparer, on procède, dans une deuxième étape du procédé selon l'invention, à l'élimination des acides aminés peu solubles dans l'eau, comme la cystine, qui risquerait de perturber les opérations ultérieures de fractionnement; cette élimination s'effectue par tout moyen convenable à la portée de l'homme de l'art tels que précipitation, cristallisation, filtration ou concentration suivi d'un refroidissement.

Dans une troisième étape, on fait subir à la solution débarrasée des fractions d'acides aminés aromatiques et basiques et de la fraction peu soluble consistant essentiellement en cystine, une nouvelle série de percolations successives sur colonnes.

Pour assurer la fixation sans fuite des acides aminés restant en solution, il serait nécessaire à ce stade d'utiliser un nombre élevé de couples de colonnes de résines anioniques et cationiques ou une colonne remplie d'un mélange de ces deux résines, ce qui est impensable sur le plan industriel car la régénération de cette dernière colonne ne serait pas possible.

Il a été découvert que la troisième étape du procédé selon l'invention comprend le passage de la solution contenant les acides aminés restants d'abord sur une colonne de résine anionique forte et ensuite sur une colonne de résine cationique forte et à recommencer au moins une fais cette double percolation sur les deux mêmes colonnes; une telle façon d'opérer, qui consiste à travailler en boucle, et où chaque passage sur colonne modifie le tampon de la solution, permet de fixer sur la résine cationique forte la fraction d'acides aminés constituée par la leucine et l'isoleucine.

Il convient de souligner qu'un tel recyclage de la solution aqueuse d'acides aminés sur un même couple formé par une colonne remplie de résine anionique et une colonne remplie de résine cationique constitue une caractéristique importante qui peut être généralisée à tout mélange d'acides aminés à séparer.

En effet, cette façon de procéder, où la solution tourne en rond en passant successivement sur deux colonnes chargées de résines échangeuses d'ions opposés, permet de faire travailler ces colonnes avec une solution dont le pH s'éloigne des zones très acides et très basiques pour se rapprocher d'une valeur voisine de la neutralité.

Au cours de ces percolations successives, le pH de la solution se stabilise à une valeur tampon qui dépend de la composition de la solution en acides aminés, et permet la fixation de certains d'entre eux sur une des colonnes en fonction de leurs points isoélectriques.

Il y a donc extraction sélective de certains acides aminés du mélange sans addition d'un milieu tampon externe par utilisation du milieu tampon interne constitué par les autres acides aminés présents dans la solution aqueuse ainsi traitée.

Dans une quatrième étape, on fait passer la solution restante de la même manière d'abord sur une colonne de résine anionique faible et ensuite sur une colonne de résine cationique forte et on recommence au moins une fois cette double percolation sur ces deux mêmes colonnes, et on constate que la colonne anionique de cette deuxième boucle fixe la fraction des acides aminés acides, à savoir les acides aspartique et glutamique et la colonne cationique de cette deuxième boucle fixe la fraction constituée par la proline, la glycine, l'alanine, la valine et la méthionine, tandis que la solution ainsi percolée contient la fraction thréonine et serine.

On réalise ainsi, selon le présent procédé, le fractionnement d'un mélange complexe de 17 acides aminés en sept fractions différentes uniquement par passage de la solution aqueuse les contenant sur des colonnes remplies de matières susceptibles de les fixer, à l'exception des opérations de précipitation de la cystine, intercalée entre plusieurs étapes de percolations.

Un tel procédé est particulièrement intéressant sur le plan industriel car chacune des fractions d'acides aminés ainsi séparée peut être récupérée pour traitement ultérieur par élution de la colonne qui l'a retenue et chacune de ces colonnes régénérée par des moyens connus de l'homme de l'art.

Ce procédé est d'autre part parfaitement adapté à la récupération des acides aminés dont les matières premières les contenant se présentent le plus souvent sous formes de quantités relativement faibles de chacun des acides aminés à séparer.

C'est notamment le cas des mélanges d'acides aminés obtenus par hydrolyse avec un acide fort de protéines, telles que des soies de porc et qui peuvent être obtenues selon le procédé faisant l'objet du brevet Français 86.04441 déposé le 27 Mars 1986 au nom de Michel FLORK.

On va maintenant décrire plus en détail le procédé selon l'invention appliqué à une solution d'hydrolyse des protéines.

Avant de soumettre la solution ou l'hydrolysat contenant le mélange d'acides aminés au procédé de l'invention, il a été découvert qu'il était préférable d'ajuster son pH et sa concentration, les niveaux choisis dépendant de la nature et du nombre d'acides aminés.

Les résultats les meilleurs ont été généralement obtenus avec un mélange de départ dont le pH est compris dans une gamme de 0,5 à 2,5 et ayant une concentration en acides aminés comprise entre 30 et 300 grammes par litre.

Dans le cas d'un hydrolysat de protéines, il faudra le plus souvent effectuer une dissolution et une neutralisation partielle, effectuée par exemple à la chaux avec séparation des sels précipités par filtration, essorage ou décantation.

Il est alors possible de soumettre la solution d'acides aminés ainsi préparée aux différentes étapes de procédé selon l'invention.

La première étape de ce procédé, qui est nouvelle en soi, consiste, comme on l'a vu ci-dessus, à soumettre la solution contenant le mélange d'acides aminés à un premier enchaînement en continue de percolations successives. La ou les premières colonnes de cet enchaînement sont des colonnes remplies de matière absorbante dites colonnes S par la suite, ladite matière consistant généralement en du charbon actif granulé. La fixation par ces colonnes des acides aminés aromatiques s'explique par l'adsorption préalable sur ces colonnes S des éléments d'acides provenant par exemple de l'acide utilisé pour l'hydrolyse des protéines.

La présence de colonnes S en tête du dispositif présente des avantages technologiques notables. Il permet d'abord la fixation ultérieure de quantités très importantes d'acides aminés sur les colonnes suivantes remplies de résines échangeuses d'ions, jusqu'à 240 kilogs par mètre cube environ. Il permet ensuite de n'utiliser que peu, voire même pas du tout, de charbon décolorant lors de la purification finale de chaque fraction ou de chacun des acides aminés séparés.

Il convient à cet égard de souligner que dans le cadre de cette première étape du procédé, le rôle de ces colonnes S ne consiste pas en un rôle banal de clarification ou de purification par filtration du mélange à traiter, mais, qu'il est beaucoup plus complexe car par suite de l'absorption purement physique de certains constituants de ce mélange par la matière absorbante dont les colonnes sont remplies, ces constituants vont pouvoir effectuer des échanges d'ions avec le reste du mélange, bien que la matière absorbante ne présente pas par elle-même de propriétés d'échanges d'ions.

On obtient de meilleurs résultats dans cette première étape en utilisant non pas une colonne S mais deux colonnes S₁ et S₂ à travers lesquelles passe successivement la solution à traiter.

La colonne amont S₁ se charge rapidement à un niveau où les fuites ne peuvent plus être évitées mais protège la colonne aval S₂ qui, peu chargée conservera plus longtemps son efficacité sans qu'apparaissent des fuites.

A la suite de ces colonnes S sont disposés un ou des couples de colonne anionique forte dite colonne A et de colonne cationique forte dite colonne C. Le couple A-C intervient pour corriger le pH, obtenir une solution-tampon et fixer certains acides aminés, en l'occurence les acides aminés basiques.

Là encore, on obtient de meilleurs résultats à ce niveau en utilisant non pas un couple de colonnes A-C mais deux couples de colonne A₁-C₁-A₂-C₂ à travers lesquelles passera successivement le mélange d'acides aminés à séparer.

On a souvent intérêt enfin à placer successivement plusieurs colonnes ayant la même fonction mais de force différente, par exemple à intercaler entre la dernière colonne S₂ et la première colonne A₁ une nouvelle colonne anionique faible dite colonne B.

L'une des mises en oeuvre préférée pour la première étape du procédé consiste donc, comme dans l'exemple 1 ci-dessous, à faire passer le mélange à séparer successivement sur la suite de colonnes disposées en série : S₁-S₂-B-A₁-C₁-A₂-C₂.

La deuxième étape du procédé selon l'invention est essentielle car l'élimination des acides aminés peu solubles dans l'eau comme la cystine est indispensable pour éviter sa cristallisation dans les colonnes, qui viendrait perturber le fractionnement des différents acides aminés par fixation dans ces dernières.

Une solution simple, pour séparer la cystine à ce stade du procédé, est de concentrer, par exemple par osmose inverse, et concentration thermique, la phase aqueuse continue, après prélèvement des acides aminés aromatiques et basiques; la cystine devient alors insoluble et se trouve en suspension; elle est donc facilement recueillie, de préférence par filtration.

On peut cependant prélever la cystine par tout autre moyen et notamment par des percolations, à condition qu'elles interviennent entre les première et troisième étapes définies ci-dessus.

Les troisième et quatrième étapes qui suivent l'élimination de la cystine sont réalisées selon les mêmes principes que la première étape par passages successifs de la solution contenant le reste des acides aminés à séparer sur une série de colonnes.

En tête du dispositif peuvent se trouver des colonnes S pour fixer les dernières quantités d'acides aminés aromatiques puis des colonnes A-C éventuellement en double.

Toutefois, le dédoublement des colonnes A-C ne suffit plus pour assurer à ce stade une fixation sans fuite des acides aminés que l'on veut prélever dans le mélange.

Pour remédier à cette difficulté, qui avait limité précédemment le recours aux résines échangeuses d'ions, on fait, selon la troisième étape du procédé de l'invention, passer le mélange à séparer sur une colonne A puis sur une colonne C et on recommence plusieurs fois cette opération avec les mêmes colonnes A et C qui travaillent en boucle.

Au cours de ce recyclage de la solution, qui tourne en rond sur un couple de colonnes A-C, chaque passage sur l'une des colonnes modifie la composition ou le pH de la solution et donc l'action de l'autre colonne.

On obtient ainsi la fixation de la fraction d'acides aminés constituée par la leucine et l'isoleucine sur la colonne C du couple A-C.

Une telle façon de procéder en boucle permet de prélever la solution en cours de traitement en un point quelconque de son parcours.

A ce stade de procédé, les derniers résidus acides, pouvant provenir notamment de l'hydrolyse acide des protéines ont été éliminées et on peut alors effectuer la quatrième étape du procédé.

Comme déjà mentionné, cette quatrième étape consiste à passe la solution sur une colonne anionique faible B puis sur une colonne cationique C et à recommencer plusieurs fois l'opération pour fixer les acides aminés acides Asp-Glu sur la première et la fraction Pro-Gly-Ala-Val-Met sur la seconde de ces colonnes de résines échangeuses d'ions, seuls les acides aminés Thr et Ser restant en solution.

Pour récupérer les acides aminés ou fractions d'acides aminés retenus dans des colonnes au cours des différentes étapes du procédé selon la présente invention, on procède à l'élution de ces colonnes et éventuellement à leur régénération.

En vue de faciliter la transposition du procédé à l'échelle industrielle, il convient de remédier aux inconvénients résultant de l'obtention de certaines fractions en solutions trop diluées.

C'est en particulier le cas lors de l'élution et de la régénération des colonnes S qui s'effectuent en une seule opération par passage d'un agent basique sur la colonne, car les régénérats obtenus sont d'abord neutres puis deviennent basiques et la plus grande partie des acides aminés aromatiques retenus dans ces colonnes se retrouvent élués dans les régénérats basiques à un niveau de dilution trop important.

Selon l'invention, l'élution-régénération d'une colonne S₁ s'effectue en prenant comme milieu d'élution les régénérats basiques provenant d'une opération précédente d'élution-régénération d'une autre colonne S₂.

En effet, une telle façon de procéder a pour effet de concentrer progressivement la solution de régénérats neutres en acides aminés aromatiques jusqu'à une teneur acceptable.

La séparation de chaque acide aminé individuel à partir d'une fraction fixée sur un colonne et qui regroupe plusieurs de ces acides peut être conduite selon toute technique appropriée.

Une de ces techniques consiste, selon l'invention, à faire passer dans la colonne une ou plusieurs solutions d'élution ou une ou plusieurs solutions de régénération qui permettent d'effectuer des prélèvements successifs des acides aminés fixés sur la colonne par modification de tampon interne et notamment par remplacement d'un milieu tampon acide aminé et acide par un milieu tampon base et acide aminé ou l'inverse.

C'est ainsi, comme on le verra dans l'exemple 2 ci-dessous, qu'une colonne de résine cationique ayant retenu un mélange d'acides aminés basiques comprenant de l'arginine, de la lysine et de l'histidine peut être éluée pour obtenir de l'arginine puis séparément un mélange constitué essentiellement de lysine et d'histidine.

Toujours selon la même technique de l'invention, on peut également prélever sur une colonne une partie des acides aminés qui y sont fixées en associant cette colonne à une colonne antagoniste, par exemple une colonne cationique à une colonne anionique et en faisant travailler ce couple de colonnes en boucle, comme expliqué ci-dessus, avec une solution d'élution convenable.

D'autre part, il est connu de procéder à la régénération tant des colonnes remplies de matière absorbante que de résine anionique par passage d'une lessive de soude puis rinçage final à l'eau pour éliminer le sodium.

Toutefois, il est difficile d'éliminer la soude par suite de sa viscosité et de son absorption sur les grains de matière absorbante ou de résine et un temps de rinçage final très long est souvent nécessaire pour y parvenir.

Pour remédier à cet inconvénient, on peut, selon l'invention, effectuer l'opération finale de rinçage lors de la régénération d'une colonne S ou A ou B, en faisant travailler cette colonne en boucle avec une colonne antagoniste, à savoir une colonne C remplie de résine cationique, qui fixe le sodium et élimine ainsi le sodium contenu dans l'eau de rinçage.

C'est ainsi qu'une colonne anionique forte A traitée par une lessive de soude nécessite normalement le passage de 14 fois son volume d'eau pendant 8 heures pour obtenir un éluat contenant moins de 100 ppm de sodium alors que cette même colonne couplée avec une colonne cationique forte C ne nécessite plus que le passage de 2 fois son volume d'eau en 4 heures pour obtenir un éluat contenant moins de 5 ppm de sodium.

Une autre caractéristique importante du procédé selon la présente invention consiste à traiter au préalable, au moins une, et de préférence chacune, des colonnes remplies soit de matière absorbante (S), soit de résine anionique (A ou B) soit de résine cationique, avec un agent oxydant.

Ce traitement consiste à remplir la colonne à traiter avec un mélange oxydant liquide convenable, comme l'eau de javel, l'eau oxygénée ou une solution de perborate et à chauffer légèrement pour amorcer une réaction exothermique de combustion des impuretés organiques en limitant l'échauffement à une température ne dépassant pas 50 à 60°C.

Au cours de cette oxydation, on oxyde, outre les impuretés, les résidus de monomères pouvant être présents dans les colonnes avec formation de gaz carbonique et on élimine les fonctions amines primaires des résines anioniques qui interfèrent avec les propriétés sélectives d'échange d'ions de ces dernières.

Grâce à ce traitement préalable de regénération du type Red/Ox, qui vient en sus du traitement classique de regénération de type pH des colonnes de résines anioniques ou cationiques et de leur rinçage final par travail en boucle avec une colonne antagoniste, on peut multiplier par cinq la quantité d'un acide aminé comme l'arginine fixée par une telle colonne.

L'ensemble du procédé de séparation d'acides aminés selon la présente invention va maintenant être illustré dans les exemples suivants par la préparation d'au moins dix acides aminés ou plus par prélèvements successifs d'au moins sept fractions à partie d'un même hydrolysat sulfurique de soies de porc.

Le premier exemple concerne la première étape de percolation en continu, le second la production d'arginine à partir des éluats ammoniacaux de la colonne C₁, le troisième la dernière percolation en boucle, le quatrième la séparation de la fraction thréonine-sérine et le cinquième le traitement préalable d'une colonne par un agent oxydant.

### Exemple 1

On effectue l'hydrolyse de soie de porc selon le procédé décrit dans la demande de brevet Français 86.04441 déposée le 27 Mars 1986 au nom de Michel FLORK en chauffant préalablement dans une cuve une solution d'acide sulfurique au moins 12N à une température d'au moins 100°C, en ajoutant simultanément la soie de porc et la quantité complémentaire d'acide sulfurique nécessaire à la neutralisation des fonctions amines des acides aminés provenant de l'hydrolyse des protéines de la soie et en arrêtant l'hydrolyse par dilution.

On ajuste le pH de l'hydrolysat ainsi obtenu à 0,8 par un apport de chaux et les sulfates précipités qui se forment sont éliminés par un filtre à bande.

La solution d'hydrolyse, ainsi neutralisée, est alors diluée jusqu'à Brix 10, ce qui correspond à une teneur en acides aminés d'environ 150 grammes par litre.

L'indice Brix d'une solution correspond au dixième de la teneur en sucre, exprimée en grammes par litre, qu'aurait une solution de saccharose dans l'eau provoquant la même réfraction de la lumière blanche.

On fait passer cette solution par percolation en continu sur des colonnes disposées en série S₁-S₂-B-A₁-C₁-A₂-C₂ qui sont toutes conformes aux dispositions préconisées par la demande de brevet français 85.17898 déposée le 27 Novembre 1985 par Michel FLORK pour assurer un écoulement homocinétique.

Dans le cas de l'exemple industriel retenu les dimensions de la virole cylindrique des colonnes sont respectivement de 1,45 mètres pour le diamètre et de 3,50 mètres pour la hauteur et le volume de matière chargée dans chaque colonne est de 5 m³.

Les colonnes S₁ et S₂ sont remplies de granulats de charbon actif, la colonne B de résine anionique faible, les colonnes A₁ et A₂ de résine anionique forte et les colonnes C₁ et C₂ de résine cationique forte.

Les colonnes ayant été préalablement régénérées et lavées par passage de la solution de lavage en boucle sur une colonne antagoniste, on y fait passer 15 m³ de la solution d'hydrolyse neutralisée et diluée que l'on pousse dans la série de colonnes avec 50 m³ d'eau pour recueillir à la sortie 20 m³ d'eau puis 45 m³ de percolats (à Brix 3 environ) qui sont concentrés à Brix 20 pour provoquer la mise en suspension de la cystine.

La colonne S₁ est régénérée en faisant passer successivement à travers elle 9 m³ de régénérats basiques provenant d'une régénération précédente puis 600 litres de NaOH 2 N et enfin 12 m³ d'eau, cette dernière tournant en boucle avec une colonne antagoniste. On obtient alors 5 m³ de régénérats neutres qui contiennent la fraction Tyr-Phe et 16 m³ de régénérats basiques que l'on concentre à 9 m³ pour les utiliser dans une opération suivante de régénération.

Une telle façon de procéder à la régénération de la colonne S₁ n'est à effectuer grâce à l'invention qu'une fois toutes les quatre percolations.

Après cette régénération de S₁, les colonnes S₁ et S₂ peuvent être permutées.

La colonne C₁ est éluée avec la première moitié des éluats de l'élution précédente concentrée à 20 m³ puis avec 5 m³ de NH₄OH 0,5N, puis avec la seconde moitié des éluats de l'élution précédente également concentrée à 20 m³ et enfin avec 5 m³ de NH₄OH 5N.

Après quatre recyclages, on recueille les éluats 5N pour la fabrication de l'arginine (voir exemple 2).

La colonne C₁ est régénérée après chaque élution par passage des régénérats acides de l'opération de régénération précédente concentrés à 15 m³ puis de 1 m³ de SO₄H₂ 6N et enfin de 10 m³ d'eau. A la sortie, on récupère environ 6 m³ de solution de sulfate d'ammonium à usage agricole et des régénérats acides à recycler dans l'opération suivante de régénération.

Après régénération de la colonne C₁, les colonnes C₁ et C₂ peuvent être permutées.

Les colonnes B,A₁ et A₂ sont régénérées en série avec 20 m³ de régénérats basiques de la régénération précédente puis 1 m³ de NaoH 9N et enfin 10 m³ d'eau. A la sortie, on recueille d'abord 11 m³ de régénérats neutres qui sont mis au rebut et dirigés vers une station d'épuration puis 20 m³ de régénérats acides qui sont recyclés dans l'opération suivante de régénération de ces colonnes.

### Exemple 2

Après quatre recyclages, les 20 m³ d'éluats 5N de la colonne C₁ sont concentrés à 6 m³ pour éliminer l'ammoniaque. La solution à Brix 12 environ est déjà relativement riche en Arg car l'autre partie de l'élution (0,5 N) a entraîné une partie notable des autres acides aminés basiques Lys et His. Il suffit de faire passer ces 6 m³ sur une colonne anionique A en les poussant avec 15 m³ d'eau. La colonne A retient diverses impuretés ainsi que les restes de Lys et His. La solution ayant traversé la colonne A est alors concentrée à Brix 50 à une température de 50°C; l'Arg précipite au refroidissement et il suffit d'une simple filtration, sans adjonction d'agent décolorant, pour obtenir l'Arg. pure.

### Exemple 3

Après passage de la solution débarrassée de la cystine selon l'exemple 1, sur un couple de colonnes A-C, travaillant en boucle, on recueille une solution de Brix 3 à 4 par lots de 5 m³.

On fait passer ces différents lots sur un couple de colonne absorbante anionique faible B et de colonne cationique forte C, travaillant en boucle avec un temps de rotation d'environ une heure par boucle.

Cette opération est répétée 3 ou 4 fois.

L'ensemble de ces opérations étant terminé, la phase liquide ne contient plus d'ions sulfatés et les acides aminés les plus acides Asp et Glu sont fixés sur la colonne B tandis que les plus basiques Pro, Gly, Ala, Val et Met sont retenus sur la colonne cationique C, seuls Thr et Ser restant dans les percolats.

### Exemple 4

La fraction Thr,Ser de l'exemple 3 se trouvant en solution dans le percolat est simplement traitée sur une colonne adsorbante de résine anionique faible B qui fixe Thr et laisse s'écouler Ser.

Cet exemple 4 illustre l'aptitude d'une même résine échangeuse d'ion à opérer une action sélective différente selon les caractéristiques de la solution percolée.

Lors de sa première utilisation dans la série de percolations de l'exemple 1, la colonne B retient les ions sulfatés et des colorants mais laisse passer tous les acides aminés.

Dans la boucle B-C de l'exemple 3, la même colonne B retient Asp et Glu mais laisse passer les autres acides aminés y compris Thr.

Enfin dans le présent exemple, cette même colonne B finit par se trouver dans les conditions voulues pour retenir Thr.

### Exemple 5

Cet exemple a pour but d'illustrer le traitement préalable d'un colonne remplie de matière absorbante ou de résine échangeuse d'ion par un agent oxydant.

En effet l'utilisation répétée d'une telle colonne provoque la rétention par cette dernière d'impuretés qui s'accumulent et viennent en diminuer fortement l'efficacité.

Les molécules d'impuretés emprisonnées à l'intérieur des grains de matière absorbante ou de résine échangeuse d'ion ne peuvent plus en ressortir et, dans la technique courante, nécessitent le remplacement des résines après quelques mois d'utilisation.

C'est ainsi qu'une colonne chargée de résine ammonium quaternaire neuve est jaune clair et devient brun foncé après de nombreuses percolations.

Sur une colonne contenant 5m³ de volume utile d'une telle résine échangeuse d'anions, on fait passer 5m³ d'une solution industrielle d'hypochlorite de sodium à 47,5 degrés chlorométrique diluée au quart donc présentant un degré chlorométrique de 12.

Au cours de cette percolation qui dure une heure, la température s'élève jusqu'à 60° environ.

On élimine l'hypochlorite pouvant rester dans la colonne en faisant passer ensuite 5m³ d'eau puis on régénère la colonne par une lessive de soude de manière connue et on obtient à nouveau une résine de couleur jaune pâle.

La colonne ayant subi un tel traitement préalable avec cet agent oxydant présente une capacité de fixation des acides aminés équivalente voire même supérieure à celle de la même colonne chargée de résine neuve, car cette dernière contient toujours quelques impuretés et des monomères.

C'est la raison pour laquelle il peut être intéressant d'effectuer ce traitement oxydant préalable des colonnes selon l'invention même si la colonne est remplie de résine neuve.

Il a été trouvé qu'une colonne chargée de résine échangeuse d'ion subissant un tel traitement une fois toutes les 250 percolations a une durée de vie qui passe de 3 mois à 7 ans.

## Revendications

1. Procédé de séparation, au moyen de résines échangeuses d'ions, d'acides aminés en mélange dans une solution aqueuse par passage de la solution sur une colonne de résine anionique puis une colonne de résine cationique, ou inversement, caractérisé par au moins un renouvellement du passage de la même solution sur ce même couple des mêmes colonnes disposées dans le même ordre et le fait que les passages successifs sur les colonnes se font sans aucun apport des substances nouvelles, l'évolution de l'action sélective d'une colonne résultant uniquement de la modification de la composition de la solution par l'échange d'ions sur la colonne antagoniste.

2. Procédé selon la revendication 1, caractérisé par le passage de la solution sur un ensemble de colonnes comprenant des colonnes de résines anioniques et cationiques disposées selon la revendication 1 et éventuellement d'autres colonnes de résines anioniques ou cationiques ou de matières adsorbantes, les passages successifs de cette solution sur toutes ces colonnes se faisant sans aucun apport de substances nouvelles, l'ajustement de l'action sélective d'une colonne résultant uniquement de la modification de la composition de la solution provoquée par l'action des colonnes précédentes.

3. Procédé selon l'une quelconque des revendications 1 ou 2 caractérisé en ce que le mélange d'acides aminés est une solution aqueuse basique, provenant notamment des jus sucrés de l'industrie de la betterave.

4. Procédé selon l'une quelconque des revendications 1 ou 2 , caractérisé en ce que le mélange d'acides aminés est une solution aqueuse acide, provenant notamment de l'hydrolyse des protéines.

5. Procédé selon la revendication 4, caractérisé en ce que la solution acide en question passe, au préalable, successivement sur des colonnes disposées en série et comprenant au moins une colonne remplie de matière adsorbante, ensuite une colonne remplie de résine anionique et enfin une colonne remplie de résine cationique.

6. Procédé selon la revendication 5, caractérisé en ce qu'on utilise comme colonne remplie de matière adsorbante (S) au moins une colonne remplie de charbon actif granulé destinée à retenir les acides aminés aromatiques Tyr et Phe.

7. Procédé selon l'une quelconque des revendications 5 ou 6, caractérisé en ce qu'après passage sur au moins une colonne remplie de matière adsorbante (S), la solution contenant les acides aminés passe d'abord sur une colonne de résine anionique faiblement basique (B), puis sur une colonne de résine anionique fortement basique (A) et ensuite sur une colonne de résine cationique fortement acide (C₁) pour retenir sur cette dernière les acides aminés basiques Hist, Lys et Arg, et enfin éventuellement à nouveau sur une seconde colonne de résine anionique fortement basique (A₂) et une seconde colonne de résine cationique fortement acide (C₂).

8. Procédé selon l'une quelconque des revendications 5 à 7, caractérisé en ce qu'après passages successifs sur des colonnes remplies de matière adsorbante (S), et de préférence également sur celles remplies de résine échangeuse d'anions (A ou B) et de résine échangeuse de cations (C), on procède à la séparation des acides aminés, peu solubles dans l'eau comme la Cys par tous moyens connus et, notamment par concentration et refroidissement, précipitation, filtration ou simple décantation.

9. Procédé selon la revendication 5 en combinaison avec l'une quelconque des revendications 6 à 8, caractérisé en ce qu'après passage de la solution contenant le mélange d'acides aminés successivement sur des colonnes remplies de matière adsorbante (S), de résine échangeuse d'anions (A ou B) et de résine échangeuse de cations (C) et précipitation des acides aminés peu solubles dans l'eau, on fait passer la solution sur une colonne remplie de résine échangeuse d'anions fortement basique (A) puis sur une colonne échangeuse de cations fortement acide (C) et on renouvelle au moins une fois le passage de la solution sur ces deux mêmes dernières colonnes (A et C), pour fixer les acides aminés Leu et Ile sur cette dernière.

10. Procédé selon la revendication 9 caractérisé en ce qu'on fait passer ensuite la solution sur une colonne remplie de résine échangeuse d'anions faiblement basique (B) puis sur une colonne échangeuse de cations fortement acide (C) et on renouvelle au moins une fois le passage de la solution sur ces deux mêmes dernières colonnes (B et C) pour fixer les acides aminés Asp et Glu sur la première (B) et les acides aminés neutres Pro, Gly, Ala, Val et Met sur la seconde (C), seuls restant en solution les acides aminés Thr et Ser.

11. Procédé selon la revendication 4 caractérisé en ce que la solution contenant le mélange d'acides aminés est constitué par le produit de l'hydrolyse de protéines par un acide minéral fort, ce produit d'hydrolyse étant de préférence à une concentration comprise entre 30 et 300 grammes par litre à un pH compris entre 0,5 et 2,5.

12. Procédé selon l'une quelconque des revendications précédentes, caractérisé en ce qu'au moins une, et de préférence chacune, des différentes colonnes remplies soit de matière adsorbante (S), soit de résine anionique (A ou B), soit de résine cationique (C) est traitée préalablement par un agent oxydant, qui peut être notamment l'eau de Javel, l'eau oxygénée ou un perborate.

## Claims

1. A process for the separation, using ion exchange resins, of a mixture of amino acids in an aqueous solution by the passage of said solution through a column of anionic resin, then a column of cationic resin, or conversely, characterized by at least one further passage of the same solution through the same pair of columns disposed in the same order, the successive passages through the columns being performed without any addition of fresh substances, and the evolution of the selective action of a column resulting solely from the modification of the composition of the solution by ion exchange on the opposed column.

2. A process according to claim 1, characterized by the passage of the solution through an assembly of columns comprising columns of anionic and cationic resins disposed according to claim 1 and possibly other columns of anionic or cationic resins or of adsorbent substances, the successive passages of the solution through all said columns being performed without any addition of fresh substances, the adjustment of the selective action of a column resulting solely from the modification of the composition of the solution caused by the action of the preceding columns.

3. A process according to either of claims 1 or 2, characterized in that the mixture of amino acids is a basic aqueous solution resulting more particularly from the sugar juices of the beet industry.

4. A process according to either of claims 1 or 2, characterized in that the mixture of amino acids is an acid aqueous solution resulting more particularly from the hydrolysis of proteins.

5. A process according to claim 4, characterized in that the acid solution is first passed through columns disposed in series and comprising at least one column filled with adsorbent material, then a column filled with anionic resin, and finally a column filled with cationic resin.

6. A process according to claim 5, characterized in that the column (S) filled with adsorbent material comprises at least one column filled with activated carbon granules adapted to retain the aromatic amino acids Tyr and Phe.

7. A process according to either of claims 5 or 6, characterized in that after passage through at least one column (S) filled with adsorbent material, the solution containing the amino acids passes first through a column (B) of weakly basic anionic resin, then through a column (A) of strongly basic anionic resin and then through a column (C₁) of strongly acidic cationic resin in order to retain thereon the basic amino acids Hist, Lys and Arg, and finally possibly through a second column (A₂) of strongly basic anionic resin and a second column (C₂) of strongly acidic resin.

8. A process according to any of claims 5 to 7, characterized in that after successive passage through columns (S) filled with adsorbent material, and preferably also through the columns (A or B, C) filled with anion exchange resins and cation exchange resins, the separation of the amino acids, such as Cys, which have low water solubility is performed by any known means and more particularly by concentration and cooling, precipitation, filtration or simple decantation.

9. A process according to claim 5 in combination with any of claims 6 to 8, characterized in that after the solution containing the mixture of amino acids has successively passed through columns (S) filled with adsorbent material, columns (A or B) filled with anion exchange resin and a column (C) filled with cation exchange resin and the amino acids of low water solubility have been precipitated, the solution is passed through a column (A) filled with strongly basic anion exchange resin and then a column (C) filled with strongly acidic cationic resin, and the solution is again passed at least once through the same last two columns (A and C) to fix the amino acids Leu and Ile thereon.

10. A process according to claim 9, characterized in that the solution is then passed through a column (B) filled with weakly basic anion exchange resin and then through a strongly acidic cation exchange column (C), and the solution is again passed at least once through the same last two columns (B and C) to fix the amino acids Asp and Glu on the first column (B) and the neutral amino acids Pro, Gly, Ala, Val and Met on the second column (C), only the amino acids Thr and Ser remaining in solution.

11. A process according to claim 4, characterized in that the solution containing the mixture of amino acids is formed by the product of the hydrolysis of proteins by a strong mineral acid, the hydrolysis product preferably having a concentration of between 30 and 300 grams per litre and a pH of between 0.5 and 2.5.

12. A process according to any of the preceding claims, characterized in that at least one, and preferably each of the different columns filled either with adsorbent substance (S) or with anionic resin (A or B) or with cationic resin (C) is pretreated with an oxidizing agent, which can more particularly be a bleach , oxygenated water or a perborate.

## Patentansprüche

1. Verfahren zur Trennung von Aminosäuren in Mischung in einer wäßrigen Lösung durch Passage der wäßrigen Lösung über eine Säule eines anionischen Harzes und dann über eine Säule eines kationischen Harzes oder umgekehrt, gekennzeichnet durch mindestens eine erneute Passage derselben Lösung über dasselbe Paar derselben, in derselben Reihenfolge angeordneten Säulen und die Tatsache, daß die aufeinander folgenden Passagen über die Säulen ohne irgendwelche Zugabe neuer Substanzen erfolgen, wobei die Entwicklung der selektiven Wirkung einer Säule allein aus der Modifikation der Zusammensetzung der Lösung durch Ionenaustausch über der Antagonistensäule resultiert.

2. Verfahren nach Anspruch 1, gekennzeichnet durch die Passage der Lösung über eine Gesamtheit von Säulen, umfassend die Säulen anionischer Harze und kationischer Harze gemäß Anspruch 1 und fakultativ andere Säulen anionischer oder kationischer Harze oder adsorbierender Materialien, wobei diese aufeinanderfolgenden Passagen dieser Lösung über alle Säulen ohne irgendeine Zugabe neuer Substanzen erfolgen und die Einstellung der selektiven Wirkung einer Säule allein aus der Modifikation der Zusammensetzung der Lösung resultiert, die durch die Wirkung der vorhergehenden Säulen herbeigeführt wurde.

3. Verfahren nach irgendeinem der Ansprüche 1 bis 3, dadurch gekennzeichnet, daß die Mischung von Aminosäuren eine wäßrige basische Lösung ist, die insbesondere aus Zuckersäften der Runkelrübenindustrie stammt.

4. Verfahren nach irgendeinem der Ansprüche 1 oder 2, dadurch gekennzeichnet, daß die Mischung von Aminosäuren eine wäßrige saure Lösung ist, die insbesondere aus der Hydrolyse von Proteinen stammt.

5. Verfahren nach Anspruch 4, dadurch gekennzeichnet, daß die fragliche saure Lösung zuvor nacheinander über Säulen läuft, die in Reihe angeordnet sind und mindestens eine mit adsorbierendem Material gefüllte Säule, dann eine mit anionischem Harz gefüllte Säule und dann eine mit kationischem Harz gefüllte Säule umfassen.

6. Verfahren nach Anspruch 5, dadurch gekennzeichnet, daß man als mit adsorbierendem Material gefüllte Säule (S) mindestens eine Säule verwendet, die mit granulierter Aktivkohle gefüllt ist und die aromatischen Aminosäuren Tyr und Phe zurückhalten soll.

7. Verfahren nach irgendeinem der Ansprüche 5 oder 6, dadurch gekennzeichnet, daß nach der Passage über mindestens eine mit adsorbierendem Material gefüllte Säure (S) die die Aminosäuren enthaltende Lösung dann über eine Säule eines schwach basischen anionischen Harzes (B), dann über eine Säule eines stark basischen anionischen Harzes (A) und dann über eine Säule eines stark sauren kationischen Harzes (C₁) läuft, um auf dieser letzteren die basischen Aminosäuren Hist, Lys und Arg zurückzuhalten, und sie schließlich möglicherweise erneut über eine zweite Säule eines stark basischen anionischen Harzes (A₂) und eine zweite Säule eines stark sauren kationischen Harzes (C₂) läuft.

8. Verfahren nach irgendeinem der Ansprüche 5 bis 7, dadurch gekennzeichnet, daß man nach den aufeinanderfolgenden Passagen über die mit adsorbierendem Material gefüllte Säule (S) und vorzugsweise auch über die mit Anionenaustauscherharz gefüllten Säulen (A oder B) und Kationenaustauscherharz gefüllte Säule (C) die in Wasser weniger löslichen Aminosäuren, wie Cys, durch jedes bekannte Mittel, insbesondere durch Konzentration und Abkühlen, Ausfällen, Filtrieren oder einfaches Dekantieren, abtrennt.

9. Verfahren nach Anspruch 5 in Kombination mit einem der Ansprüche 6 bis 8, dadurch gekennzeichnet, daß man nach der aufeinanderfolgenden Passage der die Mischung von Aminosäuren enthaltenden Lösung über die mit adsorbierendem Material gefüllte Säule (S), die mit Anionenaustauscherharz gefüllten Säulen (A oder B) und die mit Kationenaustauscherharz gefüllte Säule (C) und der Ausfällung der in Wasser wenig löslichen Aminosäuren die Lösung über eine Säule (A), die mit stark basischem Anionenaustauscherharz gefüllt ist, dann über eine mit stark saurem Kationenaustauscherharz gefüllte Säule (C) leitet und die Passage der Lösung über diese beiden letztgenannten Säulen (A und C) mindestens einmal wiederholt, um die Aminosäuren Leu und Ile auf diesen letzteren zu fixieren.

10. Verfahren nach Anspruch 9, dadurch gekennzeichnet, daß man anschließend die Lösung über eine mit schwach basischem Anionenaustauscherharz gefüllte Säule (B), dann über eine Säule eines stark sauren Kationenaustauscherharzes (C) leitet und mindestens einmal die Passage der Lösung über diese beiden letztgenannten Säulen (B und C) wiederholt, um die Aminosäuren Asp und Glu auf der ersten (B) und die neutralen Aminosäuren Pro, Gly, Ala, Val und Met auf der zweiten (C) zu fixieren, wobei nur die Aminosäuren Thr und Ser in Lösung verbleiben.

11. Verfahren nach Anspruch 4, dadurch gekennzeichnet, daß die die Mischung von Aminosäuren enthaltende Lösung aus dem Produkt der Hydrolyse von Proteinen durch eine starke Mineralsäure besteht, wobei dieses Hydrolyseprodukt vorzugsweise eine Konzentration zwischen 30 und 300 g/l bei einem pH-wert zwischen 0,5 und 2,5 hat.

12. Verfahren nach irgendeinem der vorhergehenden Ansprüche, dadurch gekennzeichnet, daß mindestens eine und vorzugsweise jede der verschiedenen mit adsorbierendem Material (S), mit anionischem Harz (A oder B) oder mit kationischem Harz (C) gefüllten Säulen vorher mit einem Oxidationsmittel behandelt wurde, das insbesondere Eau de Javel, oxygeniertes Wasser oder ein Perborat sein kann.
